# EUROPÄISCHE PATENTSCHRIFT

(11) **EP 3 158 960 B1**
(45) Veröffentlichungstag und Bekanntmachung des Hinweises auf die Patenterteilung: **18.10.2023**
(21) Anmeldenummer: 15003024.5
(22) Anmeldetag: 22.10.2015
(51) Int. Cl.: A61B 18/12, A61B 5/24, A61B 5/00

(54) **ELEKTRODE MIT DROSSEL ZUM VERHINDERN VON DURCH HF-LECKSTROM INDUZIERTEN VERBRENNUNGEN**
ELECTRODE WITH A THROTTLE FOR PREVENTING BURNS INDUCED BY HF LEAK CURRENTS
ÉLECTRODE AYANT UN ETRANGLEMENT DESTINE A EMPECHER DES COMBUSTIONS INDUITES PAR UN COURANT DE FUITE HF

(43) Veröffentlichungstag der Anmeldung: 26.04.2017
(73) Patentinhaber: inomed Medizintechnik GmbH, 79312 Emmendingen (DE)
(72) Erfinder: Baag, Matthias, 15827 Blankenfelde (DE); Krüger, Thilo, 79312 Emmendingen (DE); Mattmüller, Rudi, 79312 Emmendingen (DE); Wegner, Celine, 79106 Freiburg (DE); Wipfler, Jörg, 79312 Emmendingen (DE)
(74) Vertreter: Börjes-Pestalozza, Henrich

(56) Entgegenhaltungen:
- EP-A1- 2 314 244
- EP-A1- 2 597 481
- EP-B1- 0 057 213
- WO-A1-00/65994
- WO-A1-90/06079
- US-A- 5 782 241
- US-A1- 2008 012 568
- US-A1- 2009 054 804
- US-A1- 2012 265 045

## Beschreibung

Die Erfindung betrifft zum einen eine Elektrode, eingerichtet zur Anwendung am Körper eines Lebewesens, mit einem Körperkontaktelement zum Anlegen der Elektrode an ein Gewebe, mit einem mit dem Körperkontaktelement verbundenen Handstück zum Greifen der Elektrode.

Eine in der modernen Medizin weitreichend eingesetzte Operationsmethode ist die Hochfrequenz-Chirurgie (die auch als HF-Chirurgie, Diathermie oder Elektrokauterisation bezeichnet wird): Ein wesentlicher Vorteil dieser Operationsmethode, beispielsweise gegenüber einer herkömmlichen Verwendung eines Skalpells, besteht darin, dass gleichzeitig mit dem Schnitt eine Blutungsstillung durch einen Verschluss von zerstörten Gefäßen erfolgt.Technisch wird dies dadurch umgesetzt, dass ein Wechselstrom mit hoher Frequenz (ab etwa 300 kHz) mittels einer HF-Elektrode durch den Körper geleitet wird, um Gewebe mittels einer gewollten, durch eine hohe Stromdichte hervorgerufenen Hitzeentwicklung an der Eintrittsstelle gezielt durch Denaturierung zu schneiden und/oder zu koagulieren. Um eine ungewollte Schädigung anderer Gewebebereiche des Körpers zu verhindern, ist bei monopolarer HF-Anwendung in der Regel eine großflächiger ausgeformte Neutralelektrode zum Stromaustritt am Körper angelegt, wobei die Stromdichte an der Neutralelektrode aufgrund ihrer großflächigeren Abdeckung des Körpers (im Vergleich zur HF-Elektrode) geringer ist und an dieser Stelle daher keine Verbrennungen hervorruft.

Um während eines solchen hochfrequenz-chirurgischen Eingriffs überprüfen zu können, ob die Integrität von Nerven und neuronalen Bahnen oder Gehirnfunktionen besteht beziehungsweise durch den Eingriff versehrt wurde, wird die Hochfrequenz-Chirurgie häufig in Kombination mit intraoperativen Neuromonitoring-Anwendungen, was auch als intraoperatives neurophysiologisches Monitoring bezeichnet wird (und häufig nur als IONM oder IOM abgekürzt wird): eingesetzt. Um eine kontinuierliche und gleichzeitig mit dem HF-chirurgischen Eingriff stattfindende Kontrolle der Stimulierbarkeit, beispielsweise von durch das somatische und/oder vegetative Nervensystem gesteuerten Funktionen, zu gewährleisten, werden mindestens jeweils eine, insbesondere als Nadelelektrode ausgebildete Stimulationselektrode, und Sensoren zur Messung von Signalen an den Körper angelegt.

Durch die gleichzeitige Verwendung der HF-Chirurgie in Kombination mit intraoperativen Neuromonitoring besteht das Risiko, dass ein HF-Leckstrom über die intraoperativen Neuromonitoring-Elektroden abfließen kann. Sind die intraoperativen Neuromonitoring-Elektroden beispielsweise näher an einem chirurgischen HF-Handstück als die Neutralelektrode des HF-Chirurgiegerätes angesetzt oder ist die Neutralelektrode nicht korrekt am Patienten platziert, kann ein HF-Leckstrom über die intraoperativen Neuromonitoring-Elektroden und das intraoperative Neuromonitoring-Gerät abfließen. HF-Generatoren können sowohl in mono- als auch in bipolarer Betriebsart genutzt werden. Der für das intraoperative Neuromonitoring risikoreichere Modus ist die monopolare Betriebsart, da der HF-Strom hierbei über die Neutralelektrode abfließen soll. Aufgrund der wesentlich kleineren Oberfläche der intraoperativen Neuromonitoring-Elektroden verglichen zur Neutralelektrode kann es durch HF-Leckstrom zu einer Erhitzung an intraoperativen Neuromonitoring-Elektroden sowie deren den Körper eines Lebewesensberührenden Stromleitungen (Stromanschlüssen) kommen. Ab einerTemperatur von 42°C führt dies durch die Denaturierung des Eiweißes des Gewebes zu ungewollten Verbrennungen, insbesondere Hautverbrennungen. Selbst wenn die intraoperativen Neuromonitoring-Elektroden nicht mit einem intraoperativen Neuromonitoring-Gerät verbunden sind, können durch die Anschlusskabel der intraoperativen Neuromonitoring-Elektroden Kapazitäten entstehen, die wiederum ein Abfließen von HF-Leckströmen ermöglichen, dadurch dass eine Potentialangleichung stattfindet.

Die dieser Erfindung zugrunde liegende Aufgabe besteht also darin, eine Elektrode bereitzustellen, welche die zuvor genannten Risiken einer ungewollten Verbrennung bei einem gleichzeitigen Einsatz von HF-Chirurgie-Verfahren und einer intraoperativen Neuromonitoring-Anwendung nicht aufweist.

US2008/012568, WO90/06079, US5782241, US2012/265045, WO00/65994, EP0057213 und EP2597481 beziehen sich auf den für Anspruch 1 relevanten Stand der Technik.

Diese Aufgabe wird durch die Merkmale des Anspruchs 1 gelöst. Insbesondere wird erfindungsgemäßzur Lösung der Aufgabe vorgeschlagen, dass die Elektrode zur Anwendung am Körper eines Lebewesens eingerichtet ist und ein Körperkontaktelement zum Anlegen der Elektrode an ein Gewebe, ein mit dem Körperkontaktelement verbundenes Handstück zum Greifen der Elektrode und eine Drossel zum Verhindern des Abfließens von HF-Leckströmen über die Elektrode bei der Verwendung der Elektrode in Kombination mit einem HF-Chirurgie-Verfahren aufweist.

Die nachfolgenden Definitionen allgemeiner Begriffe können einzeln, zu mehreren oder alle zum Ersetzen der allgemeinen Begriffe herangezogen werden. So werden bevorzugte Ausführungsformen der Erfindung definiert.

Die Abkürzung "HF" bezeichnet im Kontext der Anmeldung Hochfrequenz, Hochfrequenzablation und/oder Hochfrequenzstrom. Insbesondere bezieht sie sich auf Frequenzen von mindestens 250 kHz, insbesondere mindestens 300 kHz, insbesondere mindestens 350 kHz, insbesondere von mindestens 400 kHz, insbesondere von mindestens 600 kHz, insbesondere zwischen 250 kHz und 700 kHz, insbesondere zwischen 300 kHz und 600 kHz. Die maximale Leistung typischerweise eingesetzter, vorbekannter HF-Chirurgie-Geräte beträgt zwischen 200Watt und 360 Watt, wobei in der Regel zum Schneiden (Cut) eine höhere Leistung als zur Blutstillung (Koagulation, Coag) notwendig ist.

Durch die Drossel wird hierbei verhindert, dass ungewollte HF-Leckströme über die erfindungsgemäße Elektrode abfließen können, wobei es daneben allerdings gleichzeitig möglich ist, dass gewollte Arbeitsströme, insbesondere Arbeitsströme einer niedrigeren, unterhalb einer Grenzfrequenz liegenden Frequenz, über die erfindungsgemäße Elektrode, das heißt: über die Drossel, abfließen können. Dies kann durch die Einstellung einer bestimmten Impedanz der Drossel erreicht werden, wodurch ein Bandpass, insbesondere ein Tiefpass, einrichtbar ist, durch welchen ein Strom mit einer Frequenz unterhalb einer eingerichteten Grenzfrequenz annähernd ungeschwächt passieren kann und ein Strom, der über der Grenzfrequenz liegt, gefiltert, insbesondere abgeschwächt oder gesperrt, wird.

Es kann z.B. vorgesehen sein, dass das Handstück oder zumindest die Außenflächen des Handstücks aus einem isolierenden (das bedeutet nicht stromleitenden) Material, wie Kunststoff und/oder Keramik besteht. Dadurch kann ein Berühren der erfindungsgemäßen Elektrode auch bei Stromfluss durch die Elektrode erfolgen, ohne dass ein über die Elektrode abfließender Strom durch denjenigen abfließt, der die Elektrode am Handstück berührt. Es kann beispielsweise weiter vorgesehen sein, dass eine oder mehrere der Oberflächendes Handstücks derart ausgestaltet sind, dass sie im Wesentlichen ganz oder mindestens mit 50 % ihrer Fläche an einer zu durchstechenden und/oder an der Elektrode anliegenden Oberfläche eines Gewebes anlegbar ist/sind.

Unter "flächenförmig ausgebildet" ist im Kontext der Anmeldung zu verstehen, dass eine derart ausgestaltete Oberfläche des Handstücks im Wesentlichen eben ausgestaltet ist, das heißt plan oder annähernd plan, z.B. mit leichter Wellung, angerauter oder profilierter Oberfläche, Kanälen oder dergleichen, und dergestalt, dass die Fläche rundherum einen Kantenbereich aufweist, der in sich geschlossen ist. Mit anderen Worten, (im Wesentlichen, d.h. z.B. auch ein oder mehrere Ausbuchtungen und/oder Einschnürungen aufweisende) zylindrische Oberflächen, die nur an ihren Enden Kantenbereiche aufweisen und eine (im Bereich des im wesentlichen kreisförmigen Umfangs) in sich geschlossene Oberfläche aufweisen, sind ausgeschlossen.

Ein Kantenbereich kann im Falle mehrerer, insbesondere zweier, flächenförmig ausgestalteter Oberflächen des Handstücks eine größte Tiefe (gemessen insbesondere als Erstreckung senkrecht zu einer mittleren Lage der Ebene der Oberflächen) aufweisen, die geringer ist, als die längste Breite der flächenförmig ausgestalteten Oberfläche senkrecht zur gedachten geraden Fortsetzung der Längsachse der Nadel im Verbindungsbereich (= an der Stelle, in der die Nadel in das Handstück einmündet) mit dem Handstück, beispielsweise ist diese Breite drei- bis zwanzig-, wie 3,5- bis 15-, z.B. 4-: bis 12-mal so groß wie die größte Tiefe des Kantenbereichs.

Durch die Wahl eines Winkels einer relativ (insbesondere bezogen auf eine verlängert gedachte Längsachse der Nadel im außerhalb (= distal) des Punkts des Übergangs von der Nadel in das Handstück) zu einer mit dem Handstück verbundenen Nadel gelegenen Parallelen (zum Beispiel einer parallelen ersten Außenkante des Handstücks) abgeschrägten Außenkante des Handstücks, die derart abgeschrägt ist, dass sie in Richtung zur Nadel im Verbindungsbereich näher an der gedachten geraden Verlängerung der Längsachse der Nadel in das Handstück hinein gelegen ist, als in einem dem Verbindungsbereich abgewandten Bereich, kann ein definierter Einstechwinkel (beispielsweise von 5 bis 90 °, wie von 25 bis 35 °, z.B. von 30 °) für die Elektrode genauer festgelegt werden, in dem die Elektrode dann auch fixiert werden kann.

Es kann vorgesehen sein, dass das Handstück nach Einbringen der Nadel hervorsteht oder an der Oberfläche des Gewebes anliegt. Durch eine flache Ausführung des hervorstehenden Teils des Handstücks oder durch Umlegen, insbesondere durch Klappen um die Rotationsachse der eingestochenen Nadel, insbesondere bei relativ kurzen Nadeln, kann ein ungewolltes Abstehen in den Raum verhindert werden. Ebenso kann vorgesehen sein, dass sich ein zu einer Fläche, deren eine Kante eine Abschrägung aufweist, in einem annähernd senkrechten Winkel annähernd senkrechter flächiger Abschnitt anschließt. Es kann vorgesehen sein, dass ein Winkel einer derart abgeschrägten Außenkante zu einer verlängert gedachten (distalen) Längsachse der Nadel 5° bis 60°, insbesondere 10° bis 40°, insbesondere 25° bis 35°, insbesondere 30° beträgt.

Als "Drossel" bezeichnet man eine oder mehrere Spulen, beziehungsweise Induktivitäten zur Begrenzung von Strömen in elektrischen Leitungen. Durch die Verwendung von Drosseln ist es in der Elektrotechnik möglich, Energie in Form eines Magnetfeldes zwischenzuspeichern. Durch den Einsatz von Drosseln in elektrischen Leitungen kann die Impedanz (auch als Wechselstromwiderstand bezeichnet) angepasst werden und es können bestimmte Ströme gefiltert werden.

Als "HF-Leckstrom" wird ein ungewollter Stromfluss bezeichnet, welcher nicht wie vorgesehen beispielsweise über: eine Neutralelektrode eines HF-Chirurgie-Gerätes abfließt, sondern über eine andere, am Körper des Lebewesens anliegenden Stromleitung, wie beispielsweise eine Elektrode und/oder eine Kabelleitung, wie die eines intraoperativen Neuromonitoring-Geräts.

Im Kontext dieser Anmeldung umfasst der Begriff "Lebewesen" alle Tiere und Menschen. Insbesondere sind damit Säugetiere, Vögel, Amphibien, Reptilien und/oder Fische bezeichnet. Insbesondere bezieht sich der Begriff "Lebewesen" auf den Menschen und/oder andere Säugetiere. Der Begriff "Gewebe" bezieht sich auf ein Gewebe des Körpers, insbesondere auf die Oberfläche eines Gewebes, eines Lebewesens, vorzugsweise auf ein Hautgewebe, ein Muskelgewebe, ein Bindegewebe, ein Fettgewebe, einen oder mehrere Nerven, ein Gefäß und/oder ein Organ. Der Begriff "Anlegen der Elektrode" kann im Zusammenhang der Anmeldung ein Gewebe kontaktieren, insbesondere oberflächlich berühren und/oder in ein Gewebe eindringen oder einstechen, wie insbesondere subdermal eindringen oder einstechen, bedeuten, so dass ein Strom zwischen dem Gewebe und der Elektrode fließen kann. Insbesondere kann damit gemeint sein, dass zumindest das Körperkontaktelement mit dem Gewebe direkt in Kontakt tritt. Der Begriff "Stromleitung" bezeichnet im Kontext der Anmeldung alle stromleitenden Materialien, über welche grundsätzlich ein Abfließen von HF-Leckströmen möglich ist. Beispielsweise kann damit eine Kabelleitung, eine Drahtleitung, eine Metallleitung, ein Geräteteil, ein Möbelteil oder dergleichen stromleitendes Material, Flüssigkeiten und/oder kapazitive Kopplung gemeint sein.

Erfindungsgemäß wird das Körperkontaktelement der Elektrode als stromleitfähige Nadel zum Einführen, insbesondere zum subdermalen Einstechen, der Elektrode in das Gewebe ausgestaltet. Die Nadel kann massiv oder hohl ausgebildet sein. Sie kann am distalen (vom Handstück entfernten) Ende angespitzt, abgeschrägt oder stumpf sein. Die Form der Nadel selbst kann stark variieren,

sie kann beispielsweise gerade, ein- oder mehrfach gebogen, wendelförmig, insbesondere korkenzieherförmig, mit einem Haken, insbesondere am distalen Ende, mit glatter oder mit profilierter, beispielsweise mit Widerhaken versehener, Oberfläche oder in Form einer bipolaren Nadel ausgebildet sein. Der Begriff bipolare Nadel bedeutet in diesem Zusammenhang, dass eine Elektrode zwei Pole (mit Ladung unterschiedlichen Vorzeichens) enthält, wobei die Nadelspitze als ein Pol und ein übergestülptes Kanülenrohr als ein zweiter Pol ausgestaltet ist.

Erfindungsgemäß wird die Drossel in das Handstück integriert.

Zur Einstellung eines zur Lösung der Aufgabe geeigneten Bandpasses, welcher insbesondere das Abfließen von HF-Leckströmen über die erfindungsgemäße Elektrode während des Einsatzes eines HF-Chirurgie-Verfahrens verhindert, wenn das HF-Chirurgie-Verfahren vorzugsweise mit einer Frequenz zwischen 250 kHz und 700 kHZ, insbesondere zwischen 300 kHz und 600 kHZ, insbesondere bei 300, 350, 400 und/oder 600 kHz, durchführbar ist oder durchgeführt wird, kann es zweckmäßig sein,:wenn die Drossel eine Induktivität zwischen 1,0 µH und 100,0 mH, insbesondere zwischen 100,0 µH und 20,0 mH, insbesondere zwischen 1,0 mH und 10,0 mH, insbesondere zwischen 2,0 mH und 9,0 mH, insbesondere zwischen 3,0 mH und 8,0 mH, insbesondere zwischen 4,0 mH und 7,0 mH, vorzugsweise von 4,7 mH oder 6,8 mH aufweist.

Insbesondere kann es also zweckmäßig sein, wenn die Drossel derart ausgestaltet ist, dass die untere Grenzfrequenz des Bandpasses von höchstens 7000 kHz, insbesondere von höchstens 700 kHz, insbesondere von höchstens 500 kHz, insbesondere von höchstens 400 kHz, insbesondere von höchstens 350 kHz, insbesondere von höchstens 300 kHz, insbesondere von höchstens: 250 kHz, insbesondere höchstens 220 kHz, insbesondere höchstens 20 kHz, erzeugbar ist oder entsteht. Auf diese Weise kann gewährleistet werden, dass nur die Ströme im Arbeitsfrequenzbereich der erfindungsgemäßen Elektrode unterhalb der eingerichteten Grenzfrequenz durch die erfindungsgemäße Elektrode ableitbar sind. Die eingerichtete Grenzfrequenz soll derart eingerichtet sein dass diese unterhalb der Frequenz des HF-Chirurgie Verfahrens liegt.

Es kann vorgesehen sein, dass die erfindungsgemäße Elektrode zur Verwendung mit einem Intraoperativen-Neuromonitoring-Gerät zur Kontrolle der Stimulierbarkeit von durch das somatische und/oder das vegetative Nervensystem gesteuerten Funktionen, insbesondere zur Ableitung neurophysiologischer Signale und/oder zur Stimulation von durch das somatische und/oder das vegetative Nervensystem gesteuerten Funktionen ausgebildet ist. Vorzugsweise ist das Intraoperativen-Neuromonitoring-Gerät zur Kontrolle der Stimulierbarkeit von durch das somatische und/oder das vegetative Nervensystem gesteuerten Funktionen, insbesondere in der chirurgischen Anwendung, Orthopädie, HNO, Neurochirurgie und/oder Allgemeinchirurgie ausgebildet. Des Weiteren kann vorgesehen sein, dass sie mit einen Druckknopf und/oder sterilisierungsstabil ausgebildet ist.

Zum einfachen und sicheren Anlegen der erfindungsgemäßen Elektrode an einem Gewebe kann es zweckmäßig sein, wenn das Körperkontaktelement zumindest teilweise aus einem Metall oder mehreren Metallen, welche insbesondere für den Menschen zugelassenen sind, hergestellt ist und/oder dass das Körperkontaktelement eine Länge zwischen 3,0 mm und 60,0 mm und/oder einen Außendurchmesser zwischen 0,1 mm und 8,0 mm aufweist. Vorzugsweise kann die Nadel aus einem oder mehreren leitenden Materialien, wie Titan, Platin, Iridium und/oder Wolfram hergestellt sein. Die Oberfläche der Nadel kann mit einem weiteren leitfähigen Material beschichtet, insbesondere veredelt, vorzugsweise vergoldet, sein.

Es kann weiter vorgesehen sein, dass das Handstück der erfindungsgemäßen Elektrode zumindest teilweise als Spritzgussteil und/oder als Schrumpfschlauch ausgebildet ist. Bei einer vorteilhaften Ausgestaltung der erfindungsgemäßen Elektrode kann vorgesehen sein, dass die Elektrode mit zwei oder mehr Körperkontaktelementen ausgestaltet ist, vorzugsweise derart, dass die Körperkontaktelemente in dem einen Handstück integriert und/oder damit fest verbunden sind. Alternativ oder ergänzend zu dieser Ausgestaltung der Erfindung kann vorgesehen sein, dass zwischen dem einen Körperkontaktelement oder den Körperkontaktelementen und dem Handstück jeweils ein Winkel von 90°C eingeschlossen ist, insbesondere zum senkrechten Einführen des Körperkontaktelementes oder der Körperkontaktelemente, in das Gewebe. Alternativ oder ergänzend zu den ergänzenden Merkmalen der breitesten erfindungsgemäßen Ausgestaltung der Elektrode kann vorgesehen sein, dass die Längsachse des Körperkontaktelementes oder die Längsachsen der Körperkontaktelemente koaxial zur Längsachse des Handstücks ausgerichtet ist/sind.

Eine besonders vorteilhafte Ausgestaltung kann vorsehen, dass die erfindungsgemäße Elektrode zwei oder mehr Nadeln aufweist. Vorzugsweise können die Nadeln derart an der Elektrode angeordnet sein, dass die Nadeln mit dem einen Handstück verbunden und/oder in dem einen Handstück, insbesondere in einem Verbindungsbereich des Handstücks, integriert sind.

Es kann weiter vorgesehen sein, dass zwischen dem Körperkontaktelement oder den Körperkontaktelementen und dem Handstück, insbesondere der Längsachse des Handstücks, jeweils ein Winkel zwischen 5° und 90°, insbesondere zwischen 45° und 90°, insbesondere von 45° oder von 90°, eingeschlossen ist, vorzugsweise zum senkrechten Einführen der Nadel oder der Nadeln in das Gewebe, oder dass die Längsachse der Nadel oder die Längsachsen der Nadeln koaxial zur Längsachse, insbesondere als Verlängerung des Handstücks, ausgerichtet ist/sind. Eine Ausgestaltung der erfindungsgemäßen Elektrode, bei welcher zwischen der Nadel oder den Nadeln und der Längsachse des Handstücks jeweils ein rechter Winkel eingeschlossen ist, kann den Vorteil haben, dass eine derart ausgestaltete erfindungsgemäße Elektrode, durch senkrecht auf das Handstück ausgeübte Druckausübung, einfach mittels der Nadel oder den Nadeln in ein Gewebe eindrückbar ist.

Es kann vorgesehen sein, dass ein Teil der erfindungsgemäßen Elektrode, insbesondere die zumindest eine Nadel, beschichtet oder unbeschichtet ist und/oder: dass die Elektrode als Korkenzieherelektrode ausgebildet ist, vorzugsweise wobei die zumindest eine Nadel eine Korkenzieherform aufweist. Des Weiteren kann es vorgesehen sein, dass die Elektrode monopolar oder bipolar ausgeführt ist.

Aufgrund der zuvor genannten Vorteile der erfindungsgemäßen Elektrode gemäß den hierin beschriebenen und beanspruchten Ausgestaltungen, eignet sich die erfindungsgemäße Elektrode zur Verwendung in Kombination mit einem HF-Chirurgie-Verfahren am Körper eines Lebewesens.Insbesondere eignet sich die erfindungsgemäße Elektrode zur Verwendung zum Verhindern von ungewollten, durch HF-Leckströme induzierte Verbrennungen am Körper eines Lebewesens wie hierin beschrieben.

"Es kann vorgesehen sein" oder "es kann zweckmäßig sein" oder dergleichen beschreibt jeweils spezielle Ausführungsformen der Erfindung.

Die Erfindung wird::nun anhand mehrerer, illustrierender Ausführungsbeispiele näher beschrieben, ist jedoch nicht auf diese Ausführungsbeispiele beschränkt. Weitere Ausführungsbeispiele ergeben sich durch Kombination einzelner oder mehrerer Merkmale der Schutzansprucheuntereinander und/oder einzelner oder mehreren Merkmale der Ausführungsbeispiele. Solche Ausführungsformen müssen jedoch innerhalb des Schutzbereichs der beigefügten Ansprüche liegen.

Es zeigt:
- Fig.1: Ein System mit zwei erfindungsgemäßen Elektroden einer spezifischen Ausführungsform, die mit einem Intraoperativen-Neuromonitoring-Gerät verbunden sind in schematischer Darstellung,
- Fig. 2: Eine zum Teil als Schnitt durch das Handstück dargestellte, schematische Detailansicht einer in Fig. 1 abgebildeten und mit einer gestrichelten Linien umrandeten erfindungsgemäßen Elektrode,
- Fig. 3: Eine zum Teil als Schnitt durch das Handstück dargestellte, schematische: Detailansicht einer weiteren bestimmten Ausführungsform einer erfindungsgemäßen Elektrode mit zwei als Nadeln ausgebildeten Körperkontaktelementen,
- Fig. 4: Eine schematische Detailansicht einer weiteren bestimmten Ausführungsform einer erfindungsgemäßen Elektrode mit zwei als Nadeln ausgebildeten Körperkontaktelementen,
- Fig. 5: Eine zum Teil als Schnitt durch das Handstück dargestellte, schematische Detailansicht einer weiteren bestimmten Ausführungsform einer erfindungsgemäßen Elektrode mit einem als Schrumpfschlauch ausgebildeten Handstück,
- Fig. 6: Eine zum Teil als Schnitt durch das Handstück dargestellte, schematische Detailansicht einer weiteren bestimmten Ausführungsform einer erfindungsgemäßen Elektrode mit einem korkenzieherförmigen Körperkontaktelement,
- Fig. 7: Eine zum Teil als Schnitt durch ein Gehäuse dargestellte, schematische Detailansicht eines nicht erfindungsgemäßen Nachrüstelements in Draufsicht,
- Fig. 8: Eine schematische Darstellung eines unteren Teils eines Gehäuses eines nicht erfindungsgemäßen Nachrüstelements in perspektivischer Ansicht,
- Fig. 9: Eine zum Teil transparent und schematisch dargestellte, perspektivische Ansicht eines nicht erfindungsgemäßen Nachrüstelements,
- Fig. 10: Eine schematische Darstellung einesvorbekannten Systems (entsprichtdem Stand der Technik).

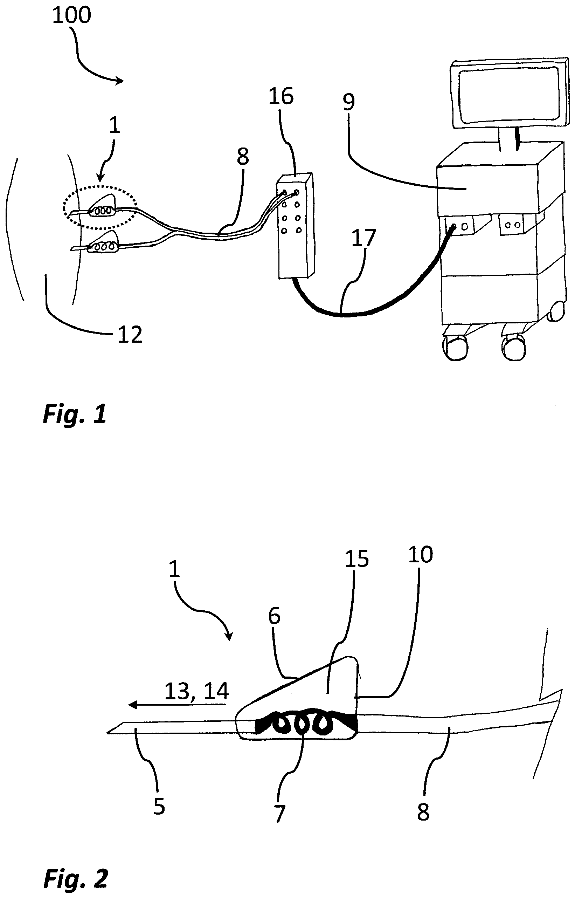

In Figur 1 ist ein System 100 mit einer bestimmten Ausführungsform einer erfindungsgemäßen Elektrode 1 dargestellt, die mittels einer Stromleitung 8 an ein Intraoperatives-Neuromonitoring-Gerät 9 zur Kontrolle der Stimulierbarkeit beispielsweise von durch das somatische und/oder vegetative Nervensystem gesteuerten Funktionen angeschlossen ist. Insbesondere kann das Intraoperative-Neuromonitoring-Gerät 9 zur Ableitung neurophysiologischer Signale und/oder zur Stimulation von durch das somatische und/oder das. vegetative Nervensystemgesteuerten Funktionen, vorzugsweise in der chirurgischen Anwendung in der Orthopädie (Rücken), HNO und Neurochirurgie (Kopf) und Allgemeinchirurgie (Rumpf und Extremitäten), wie beispielsweise insbesondere im Beckenbereich oder Beckenbodenbereich, ausgelegt sein.

In Figur 2 ist die in Figur 1 dargestellte Elektrode 1, die anhand der gestrichelten Linie umrandet ist, in einer schematischen Detailansicht abgebildet. Die Ausführungsformen der erfindungsgemäßen Elektrode 1, 2, 3, 4, 30 gemäß den Figuren 1 bis 6 weisen jeweils zumindest ein Körperkontaktelement 5 zum Anlegen der Elektrode 1, 2, 3, 4, 30an ein Gewebe 12 auf. Erfindungsgemäß ist das Körperkontaktelement 5 als eine Nadel zum Einführen, insbesondere zum Einstechen, vorzugsweise zum subdermalen Einstechen, in das Gewebe 12 ausgebildet. Das zumindest eine Körperkontaktelement 5 ist aus zumindest einem leitfähigen Material hergestellt und über eine Drossel 7 mit einer Stromleitung 8 verbunden.

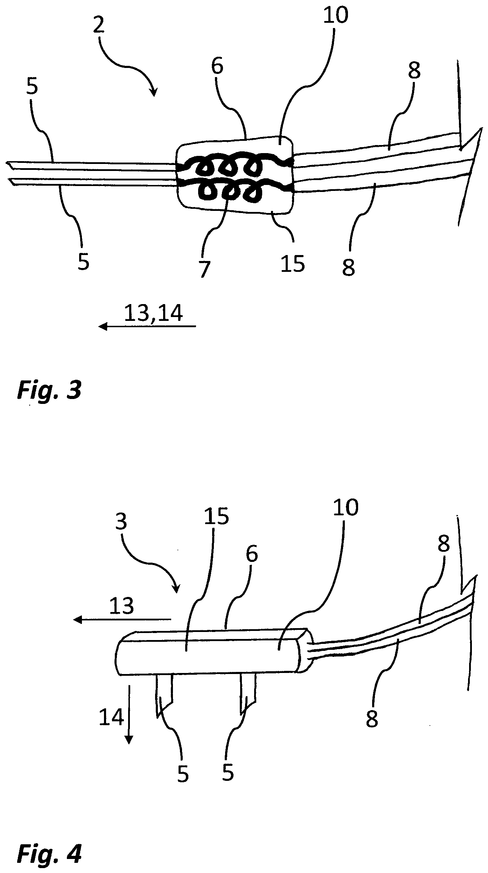

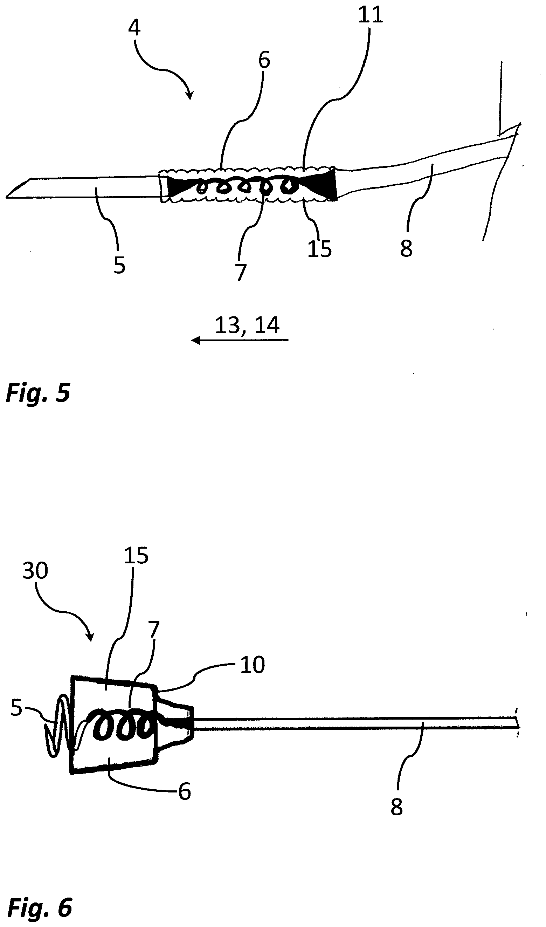

Zur einfacheren Handhabung der erfindungsgemäßen Elektrode 1, 2, 3, 4, 30 ist allgemein vorgesehen, dass diese ein mit dem Körperkontaktelement 5 verbundenes Handstück 6, geeignet zum Greifen der Elektrode 1, 2, 3, 4, 30 aufweist. Um das Abfließen von HF-Leckströmen über die erfindungsgemäße Elektrode 1, 2, 3, 4, 30 bei einer Verwendung der Elektrode 1, 2, 3, 4, 30 in Kombination mit einem HF-Chirurgie-Verfahren zu verhindern, ist vorgesehen, dass die Elektrode 1, 2, 3, 4, 30 eine Drossel 7 aufweist. Erfindungsgemäß ist die Drossel 7, wie in den Figuren 1 bis 3 sowie in Figur 5 und 6 gezeigt ist, im Inneren des Handstücks 6 eingehaust. In Figur 4 ist zwar keine Drossel dargestellt (da die Figur 4 nicht als Schnittansicht dargestellt ist). Allerdings ist auch bei dieser Ausführungsform der erfindungsgemäßen Elektrode 3 eine Drossel 7 im Inneren des Handstückes 6 vorgesehen, um das Abfließen von HF-Leckströmen über die Elektrode 1, 2, 3, 4, 30 zu verhindern. Vorzugsweise ist die Drossel 7 als Leitungszwischenstück in der Stromleitung 8 integriert oder eingesetzt oder nachträglich einsetzbar, wobei die Drossel 7 in der Stromleitung 8 in Reihe geschalten ist.

Die Stromleitung 8 zum Anschließen der erfindungsgemäßen Elektrode 1 kann als fester Bestandteil der erfindungsgemäßen Elektrode 1, 2, 3, 4, 30, insbesondere am Handstück 6 befestigt, ausgestaltet sein oder mit dem Handstück 6 über ein oder mehrere Kontaktelemente (nicht dargestellt), insbesondere über einen Stecker und eine Buchse, verbindbar sein, insbesondere derart, dass die Stromleitung 8 lösbar mit dem Handstück 6 verbindbar ist. Vorzugsweise ist die Stromleitung 8 als nach außen hin isolierte Kabelleitung ausgebildet. Vorzugsweise beträgt die Gesamtlänge der Stromleitung 8 der Elektrode, insbesondere der Stromleitung zwischen Elektrode und IOM-Gerät oder zwischen Elektrode und einer Adapterbox,zwischen 0,10m und 7,0 m, insbesondere zwischen 0,25 m und 5,0 m, insbesondere zwischen 0,5 und 4,5 m, insbesondere zwischen 0,8 m und 4,0 m, insbesondere zwischen 1,0 und 3,5 m, vorzugsweise zwischen 0,12 m und 3,0 m, vorzugsweise zwischen 1,2 m und 1,5 m.

Die Drossel 7 kann eine Induktivität zwischen 1,0 µH und 100,0 mH, insbesondere zwischen 100,0 µH und 20,0 mH, insbesondere zwischen 1,0 mH und 10,0 mH, insbesondere, zwischen 2,0 mH und 9,0 mH, insbesondere zwischen 3,0 mH und 8,0 mH, insbesondere zwischen 4,0 mH und 7,0 mH, vorzugsweise von 4,7 mH oder 6,8 mH aufweisen. Vorzugsweise ist die Drossel 7 der erfindungsgemäßen Elektrode 1, 2, 3, 4, 30 dadurch derart eingerichtet, dass ein Bandbreitenfilter zur Erzeugung einer Resonanzfrequenz von höchstens 400 kHz, insbesondere von höchstens 300 kHz, insbesondere von höchstens 250 kHz, insbesondere höchstens 220 kHz, erzeugbar ist oder entsteht.

Das Körperkontaktelement 5, welches, wie hier in den Figuren 1 bis 6 dargestellt ist, erfindungsgemäß als Nadel ausgestaltet ist, kann zumindest teilweise aus Metall hergestellt sein. Besonders geeignete Metalle zur Herstellung können beispielsweise Edelstahl und/oder Platin-Iridium sein. Ferner kann es vorgesehen sein, dass das Körperkontaktelement 5 beschichtet ist. Das als Nadel ausgeformte Körperkontaktelement 5 kann verschiedene Längen aufweisen, die an die jeweils vorgesehene Anwendung anpassbar sind. Es kann insbesondere vorgesehen sein, dass das Körperkontaktelement 5 eine Länge zwischen 3,0 mm und 60,0 mm und/oder einen Außendurchmesser zwischen 0,1 mm und 8,0 mm aufweist.

Das Handstück 6 kann zum Beispiel als ein Gehäuse ausgestaltet sein, welches beispielsweise aus Kunststoff hergestellt ist. Es kann insbesondere vorteilhaft sein, wenn zumindest ein Teil des Handstücks 6 als Spritzgussteil 10 und/oder als Schrumpfschlauch 11 (siehe Figur 5) ausgeführt ist. Es kann weiter:vorteilhaft sein, wenn das Handstück 6 eine Grifffläche 15 aufweist zum einfacheren Halten, Greifen und Platzieren der Elektrode 1, 2, 3, 4 ,30. Die Grifffläche 15 kann dabei beispielsweise als rutschfeste Fläche, vorzugsweise als gummierte Fläche, ausgeführt sein. Es kann weiterhin vorteilhaft sein, wenn die erfindungsgemäße Elektrode 1, 2, 3, 4, 30 aus Materialien hergestellt ist, die zur Sterilisation und/oder Autoklavierung geeignet sind. Ferner kann vorgesehen sein, dass die erfindungsgemäße Elektrode 1, 2, 3, 4, 30 als Einmalprodukt ausgestaltet ist, das nach Gebrauch entsorgt wird.

In den Figuren 3 und 4 sind zwei spezielle Ausgestaltungen der erfindungsgemäßen Elektrode 2, 3 dargestellt, wobei diese jeweils zwei, als Nadeln ausgeführte Körperkontaktelemente 5 aufweisen. Beide Nadeln sind dabei mit jeweils einem Handstück 6 verbunden.

Bei der in Figur 3 dargestellten Ausführungsform: der erfindungsgemäßen Elektrode 2 ist vorgesehen, dass die Längsachsen 14 der Körperkontaktelemente 5 koaxial zur Längsachse 13 des Handstücks ausgerichtet sind.

Bei der in Figur 4 dargestellten Ausführungsform der erfindungsgemäßen Elektrode 3 ist vorgesehen, dass zwischen den Körperkontaktelementen 5 und dem Handstück 6 jeweils ein Winkel von 90°C eingeschlossen ist. Das bedeutet, dass die Längsachsen 14 der Körperkontaktelemente 5 senkrecht zur Längsachse 13 des Handstücks 6 ausgerichtet sind. Diese Art der Ausrichtung ermöglicht es, dass die Elektrode 3 auf die Oberfläche eines Gewebes 12 aufgesetzt werden kann und mittels ihrer, als Nadeln ausgebildeten Körperkontaktelemente 5 durch Druckausübung auf das Handstück 6, insbesondere per Druckausübung mit einem Daumen, nahezu senkrecht in das Gewebe 12 einführbar ist.

In Figur 5 ist eine Ausführungsform der Erfindung dargestellt, bei welcher das Handstück 6 der Elektrode 4 als Schrumpfschlauch 11 ausgestaltet ist.

Bei der in Figur 6 dargestellten Ausführungsform der erfindungsgemäßen Elektrode 30 ist das Körperkontaktelement 5 als eine Nadel in einer spiralförmigen Korkenzieherform ausgestaltet, wobei die Spitze dieses Körperkontaktelements 5 abgeschrägt und spitz zulaufend ist. Das Körperkontaktelement 5 ist mit der Drossel 7 verbunden, wobei die Drossel 7 in das Handstück 6 eingehaust ist. Diese Ausgestaltung hat den Vorteil, dass die Korkenzieherelektrode durch Drehen um die Längsachse im oder gegen den Uhrzeigersinn (je nach Ausrichtung der einzelnen Windungen) einfach und in einer, durch die Spiralform definierten Richtung in ein Gewebe, insbesondere in die Haut, eindringen kann und dadurch am Gewebe, insbesonderean der Haut, anlegbar ist.

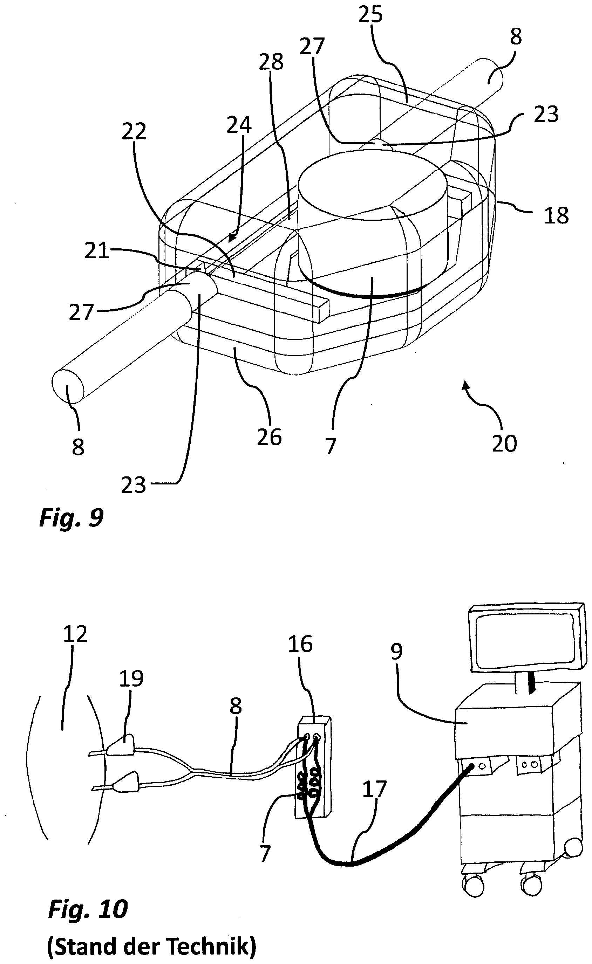

Um die wesentlichen Unterschiede der erfindungsgemäßen Elektrode 1, 2, 3, 4, 30 gegenüber konventionellen Elektroden beziehungsweise eines Systems 100 mit einer erfindungsgemäßen Elektrode 1, 2, 3, 4, 30 (in Figur 1 ist lediglich die erfindungsgemäße Elektrode 1 dargestellt, wobei auch jede andere erfindungsgemäße Ausführungsform möglich ist) gegenüber einem konventionellen System, besser verdeutlichen zu können, ist in der Figur 10 der derzeit üblicherweise zum Einsatz kommende Stand der Technik dargestellt. Wie bereits zuvor in der Beschreibung der Anmeldung erläutert wurde, besteht ein Nachteil derartiger konventioneller Elektroden 19 und Systeme aus einem Intraoperativen-Neuromonitoring-Gerät 9 und einer konventionellen Elektrode 19, die am Körper eines Lebewesens,: insbesondere eines Menschen, angelegt wird, darin, dass es beispielsweise bei einer Verwendung in Kombination mit einem HF-Chirurgie-Verfahren durch das Abfließen von HF-Leckströmen über eine Stromleitung 8 der konventionellen Elektrode 19 zu unerwünschten Verbrennungen am Gewebe 12 des Körpers kommen kann. Bei einer aus dem Stand der Technik vorbekannten und in Figur 6 abgebildeten Lösung wurde daher versucht, dieses Problem damit zu lösen, indem eine Stromleitung 17 zwischen einem, beispielsweise als Adapterbox ausgebildeten Verbindungselement 16 und einem Intraoperativen-Neuromonitoring-Gerät 9 eingesetzt wird, die eine Drossel 7 aufweist. Dadurch kann zwar möglicherweise verhindert werden, dass HF-Leckströme über die Stromleitung 17 abfließen können, allerdings kann damit oder mittels einer anderen aus dem Stand der Technik vorbekannten Lösung nicht verhindert werden, dass HF-Leckströme über eine Stromleitung 8 der konventionellen Elektrode 19 selbst abfließen und es somit dennoch zu unerwünschten Verbrennungen des Gewebes 12 kommt. Dieses Problem konnte nun erstmals durch die vorliegende Erfindung wie hierin beschrieben und beansprucht gelöst werden.

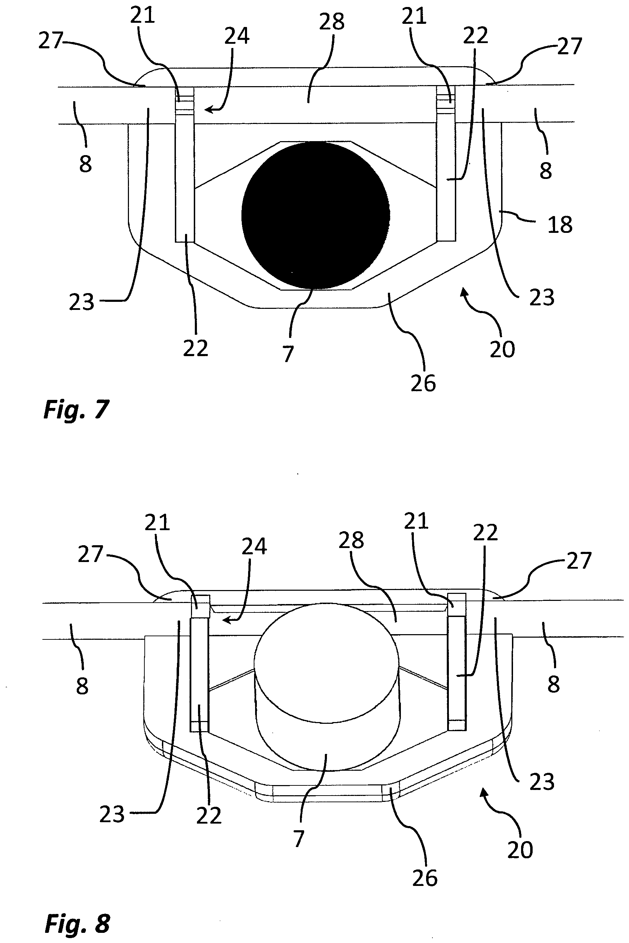

In den Figuren 7 bis 9 ist eine spezifische Ausführungsform eines nicht Z erfindungsgemäßen Nachrüstelements 20 zum Einsetzen als Leitungszwischenstück in einer Stromleitung 8 dargestellt. Das Nachrüstelement 20 weist ein Gehäuse 18 auf und ist insbesondere mittels zwei als Crimp-Anschlüsse 24 ausgebildeten Kontaktelementen 21 an zwei freie Enden 23 einer Stromleitung 8 als Leitungszwischenstück einsetzbar.

So kann beispielweise vorgesehen sein, dass ein Gehäuse 18 ein erstes Gehäuseteil 25 und ein zweites Gehäuseteil 26 aufweist, wobei es derart ausgestaltet ist, dass es von einer Offenstellung, in welcher eine durchgehende (=nicht unterbrochene) Stromleitung 8 in das Nachrüstelement 20 einsetzbar ist, in eine Schließstellung durch Zusammenfügen des ersten Gehäuseteils 25 mit dem zweiten Gehäuseteil 26 versetzbar ist.

Damit das Nachrüstelement 20 möglichst einfach in eine durchgehende Stromleitung 8 einsetzbar ist, kann vorgesehen sein, dass es eine Schneidevorrichtung 22, insbesondere eine zumindest teilweise leitfähige Schneidevorrichtung 22, zum Durchtrennen und/oder zum Entfernen eines Teils der Stromleitung 8 aufweist, wobei durch die Schneidevorrichtung 22 zwei freie Enden 23 der Stromleitung 8 erzeugbar sind, an welche wiederum die beiden Kontaktelemente 21 ansetzen, um das Nachrüstelement 20 als Leitungszwischenstück mit der Stromleitung 8 zu verbinden. Die Kontaktelemente 21 und die Schneidevorrichtung 22 können, wie in den Figuren 7 bis:9 dargestellt ist, als eine kombinierte Kontakt-Schneidevorrichtung, beispielsweise in Form einer Abschereinheit, insbesondere als ein oder mehrere, vorzugsweise zwei, stromleitfähigeAbscherbleche, ausgestaltet sein. So kann die undurchtrennte Stromleitung 8 in Offenstellung in das Nachrüstelement 20 eingelegt werden und durch Schließen des Nachrüstelement 20 kann mittels des Abscherblechs oder der Abscherbleche die Stromleitung 8 durchtrennt werden und gleichzeitig kann durch das Abscherblech oder die Abscherbleche ein Stromfluss über die Drossel 7 herstellbar sein.

Besonders vorteilhaft kann es sein, wenn das Nachrüstelement 20 als Nachrüst-Clip ausgestaltet ist, wobei die beiden Gehäuseteile 25, 26 mittels zumindest eines Scharniers relativ zueinander beweglich verbunden sein können, so dass das Nachrüstelement 20 mittels des zumindest einen Scharniers öffenbar und schließbar ist oder mittels eines Clip-Mechanismus verbindbar sind oder miteinander verbunden sind, wobei das Nachrüstelement 20 durch Zusammenclippen der beiden Gehäuseteile 25, 26 in eine Schließstellung, insbesondere reversibel, versetzbar ist. Hierbei kann es beispielsweise vorgesehen sein, dass das als Nachrüst-Clip ausgestaltete Nachrüstelement 20 eine kombinierte Schneide-Kontakt-Vorrichtung (wobei diese derart ausgestaltet sein kann, wie bereits zuvor beschrieben wurde) zum Durchtrennen und Entfernen eines abgetrennten Teils 28 der Stromleitung 8 und zum gleichzeitigen Anschließen von zwei freien Enden 23 der unterbrochenen Stromleitung aufweist.

In Schließstellung des Nachrüstelements 20 ist vorgesehen, dass ein Stromfluss lediglich durch die Drossel 7 des Nachrüstelements 20 möglich ist. Um dies zu verwirklichen, kann beispielsweise vorgesehen sein, dass durch eine beim Schließen der beiden Gehäuseteile 25, 26 erzeugte Betätigungskraft eine Schneidevorrichtung 22 betätigbar ist, wodurch die Stromleitung::8 mittels der Schneidevorrichtung 22 an zumindest einer Stelle durchtrennbar ist und somit zumindest zwei freie Enden 23 der Stromleitung 8 erzeugbar sind. Es kann ebenso vorgesehen sein, dass durch die Schneidevorrichtung 22 ein Zwischenstück oder ein Teil 28 der Stromleitung 8 herausgeschnitten wird. Somit entstehen drei Leitungsteile: ein Teil der Stromleitung 8 zum Körperkontaktelement 5, ein Teil der Stromleitung 8 zum IOM-Gerät 9 und/oder zum Verbindungselement 16 sowie das abgetrennte Leitungsstück 28, welches insbesondere in einer Wanne, insbesondere einer Kunststoffwanne, aufnehmbar ist oder durch eine Öffnung, welche als Auswurf dient, auswerfbar ist, so dass kein Strom mehr über das abgetrennte Leitungsstück 28 fließen kann. Durch zumindest zwei Klemmvorrichtungen 27, die vorzugsweise am Gehäuse 18 ausgestaltet sind, welche in Schließstellung des Gehäuses 18 den Teil der Stromleitung 8 zum Körperkontaktelement 5 und den Teil der Stromleitung 8 zum IOM-System greifen, werden die freien Enden 23 der Stromleitung 8 in dem Nachrüstelement: 20 fixiert. Die Klemmvorrichtungen 27 können beispielsweise als Durchlassöffnungen, vorzugsweise als runde oder ovale Durchlassöffnungen, am Gehäuse 18 ausgebildet sein. In Schließstellung des Nachrüstelements 20 verbinden die beiden Kontaktelemente 21 die Drossel 7 als Leitungszwischenstück der Stromleitung 8 mit den beiden freien Enden 23. Insbesondere kann vorgesehen sein, dass der Strom dann nur noch über die Abscherbleche und somit über die Drossel 7 fließt.

### Bezugszeichenliste

- 1: Ausführungsform der erfindungsgemäßen Elektrode
- 2: Ausführungsform der erfindungsgemäßen Elektrode
- 3: Ausführungsform der erfindungsgemäßen Elektrode
- 4: Ausführungsform der erfindungsgemäßen Elektrode
- 30: Ausführungsform der erfindungsgemäßen Elektrode
- 5: Körperkontraktelement
- 6: Handstück
- 7: Drossel
- 8: Stromleitung der Elektrode
- 9: Intraoperatives-Neuromonitoring-Gerät
- 10: Spritzgussteil
- 11: Schrumpfschlauch
- 12: Gewebe
- 13: Längsachse des Handstücks 6
- 14: Längsachse des Körperkontaktelements 5
- 15: Grifffläche
- 16: Verbindungselement
- 17: Stromleitung vom Verbindungselement 16 zum intraoperativen-Neuromonitoring-Gerät 9
- 18: Gehäuse
- 19:: konventionelle Elektrode (Stand der Technik)
- 20: Nachrüstelement
- 21: Kontaktelement
- 22: Schneidevorrichtung
- 23: freie Enden der Stromleitung 8
- 24: Crimp-Anschluss
- 25: erstes Gehäuseteil
- 26: zweites Gehäuseteil
- 27: Klemmvorrichtung
- 28: Abgetrenntes Leitungsstück
- 100: System

## Patentansprüche

1. Elektrode (1, 2, 3, 4, 30), eingerichtet zur Anwendung am Körper eines Lebewesens, mit einem Körperkontaktelement (5) zum Anlegen der Elektrode (1, 2, 3, 4, 30) an ein Gewebe (12), mit einem mit dem Körperkontaktelement (5) verbundenen Handstück (6) zum Greifen der Elektrode (1, 2, 3, 4, 30) und mit einer Drossel (7) in Form von einer oder mehreren Spulen zum Verhindern des Abfließens von HF-Leckströmen über die Elektrode (1, 2, 3, 4, 30) bei der Verwendung der Elektrode (1) in Kombination mit einem HF-Chirurgie-Verfahren, wobei , das Körperkontaktelement (5) als stromleitfähige Nadel zum Einführen, insbesondere zum subdermalen Einstechen, in das Gewebe (12) ausgestaltet ist und dass die Drossel (7) in das Handstück (6) integriert ist.

2. Elektrode (1, 2, 3, 4, 30) nach Anspruch 1, **dadurch gekennzeichnet, dass** die Drossel (7) eine Induktivität zwischen 1,0 µH und 100,0 mH, insbesondere zwischen 100,0 µH und 20,0 mH, insbesondere zwischen 1,0 mH und 10,0 mH, insbesondere zwischen 2,0 mH und 9,0 mH, insbesondere zwischen 3,0 mH und 8,0 mH, insbesondere zwischen 4,0 mH und 7,0 mH, vorzugsweise von 4,7 mH oder 6,8 mH aufweist und/oder dass die Drossel (7) derart ausgestaltet ist, dass ein Bandbreitenfilter zur Erzeugung einer Resonanzfrequenz von höchstens 400 kHz, insbesondere von höchstens 300 kHz, insbesondere von höchstens 250 kHz, insbesondere höchstens 220 kHz, erzeugbar ist oder entsteht.

3. Elektrode (1, 2, 3, 4, 30) nach einem der Ansprüche 1 oder 2, **dadurch gekennzeichnet, dass** die Elektrode (1, 2, 3, 4, 30) zur Verwendung mit einem Intraoperativen-Neuromonitoring-Gerät (9) zur Kontrolle der Stimulierbarkeit von durch das somatische und/oder das vegetative Nervensystem gesteuerten Funktionen, insbesondere zur Ableitung neurophysiologischer Signale und/oder zur Stimulation von durch das vegetative Nervensystem gesteuerten Funktionen, vorzugsweise in der chirurgischen Anwendung in der Orthopädie, HNO, Neurochirurgie und/oder Allgemeinchirurgie ausgebildet ist.

4. Elektrode (1, 2, 3, 4, 30) nach einem der Ansprüche 1 bis 3, **dadurch gekennzeichnet, dass** das Körperkontaktelement (5) zumindest teilweise leitfähig, vorzugsweise aus Metall, insbesondere aus Edelstahl, Platin-Iridium oder anderen Metallen, hergestellt ist und/oder dass das Körperkontaktelement (5) eine Länge zwischen 3,0 mm und 60,0 mm und/oder einen Außendurchmesser zwischen 0,1 mm und 8,0 mm aufweist.

5. Elektrode (1, 2, 3, 4, 30) nach einem der Ansprüche 1 bis 4, **dadurch gekennzeichnet, dass** das Handstück (6) zumindest teilweise als Spritzgussteil (10) und/oder als Schrumpfschlauch (11) ausgestaltet ist.

6. Elektrode (1, 2, 3, 4, 30) nach einem der Ansprüche 1 bis 5, **dadurch gekennzeichnet, dass** die Elektrode (1, 2, 3, 4, 30) mit zwei oder mehr Körperkontaktelementen (5) ausgestaltet ist, derart, dass die Körperkontaktelemente (5) mit dem einen Handstück (6) verbunden sind, und/oder dass zwischen den Körperkontaktelementen (5) und dem Handstück (6) jeweils ein Winkel von ungefähr 90°C eingeschlossen ist, insbesondere zum senkrechten Einführen der Körperkontaktelemente (5) in das Gewebe, oder dass die Längsachse (14) der Körperkontaktelemente (5) koaxial zur Längsachse (13) des Handstücks ausgerichtet sind.

7. Elektrode (1, 2, 3, 4, 30) nach einem der Ansprüche 1 bis 6, **dadurch gekennzeichnet, dass** die Nadel oder die Nadeln beschichtet oder unbeschichtet ist/sind und/oder dass die Elektrode als Korkenzieherelektrode ausgebildet ist, vorzugsweise wobei die Nadel eine Korkenzieherform aufweist, vorzugsweise wobei die Elektrode (1, 2, 3, 4, 30) monopolar oder bipolar ausgeführt ist.

## Claims

1. Electrode (1, 2, 3, 4, 30) designed for use on the body of a living being, comprising a body contact element (5) for bringing the electrode (1, 2, 3, 4, 30) to lie against a tissue (12), comprising a handpiece (6) for gripping the electrode (1, 2, 3, 4, 30), which handpiece is connected to the body contact element (5), and comprising a throttle (7) in the form of one or more coils for preventing the outflow of HF leakage currents through the electrode (1, 2, 3, 4, 30) when the electrode (1) is being used in combination with a HF surgical procedure, wherein the body contact element (5) is in the form of an electrically conductive needle for insertion, in particular for subdermal puncture, into the tissue (12) and in that the throttle (7) is integrated in the handpiece (6).

2. Electrode (1, 2, 3, 4, 30) according to Claim 1, **characterized in that** the throttle (7) has an inductance between 1.0 µH and 100.0 mH, in particular between 100.0 µH and 20.0 mH, in particular between 1.0 mH and 10.0 mH, in particular between 2.0 mH and 9.0 mH, in particular between 3.0 mH and 8.0 mH, in particular between 4.0 mH and 7.0 mH, preferably 4.7 mH or 6.8 mH, and/or **in that** the throttle (7) is designed such that a bandwidth filter for generating a resonant frequency of at most 400 kHz, in particular at most 300 kHz, in particular at most 250 kHz, in particular at most 220 kHz, can be created or is produced.

3. Electrode (1, 2, 3, 4, 30) according to either of Claims 1 and 2, **characterized in that** the electrode (1, 2, 3, 4, 30) is designed for use with an intraoperative neuromonitoring apparatus (9) for controlling the stimulability of functions controlled by the somatic and/or autonomic nervous system, in particular for generating neurophysiological signals and/or for stimulating functions controlled by the autonomic nervous system, preferably in surgical use in orthopaedics, ENT, neurosurgery and/or general surgery.

4. Electrode (1, 2, 3, 4, 30) according to one of Claims 1 to 3, **characterized in that** the body contact element (5) is at least partially conductive, preferably is produced from metal, in particular from stainless steel, platinum-iridium or other metals, and/or **in that** the body contact element (5) has a length between 3.0 mm and 60.0 mm and/or an outside diameter between 0.1 mm and 8.0 mm.

5. Electrode (1, 2, 3, 4, 30) according to one of Claims 1 to 4, **characterized in that** the handpiece (6) is at least partially in the form of an injection moulded part (10) and/or a shrink tube (11).

6. Electrode (1, 2, 3, 4, 30) according to one of Claims 1 to 5, **characterized in that** the electrode (1, 2, 3, 4, 30) is formed with two or more body contact elements (5) such that the body contact elements (5) are connected to the one handpiece (6), and/or **in that** an angle of approximately 90° is formed between each body contact element (5) and the handpiece (6), in particular for the perpendicular insertion of the body contact element (5) into the tissue, or **in that** the longitudinal axes (14) of the body contact elements (5) are aligned coaxially with the longitudinal axis (13) of the handpiece.

7. Electrode (1, 2, 3, 4, 30) according to one of Claims 1 to 6, **characterized in that** the needle or the needles is/are coated or uncoated and/or **in that** the electrode is in the form of a corkscrew electrode, preferably wherein the needle has a corkscrew shape, preferably wherein the electrode (1, 2, 3, 4, 30) has a monopolar or bipolar design.

## Revendications

1. Electrode (1, 2, 3, 4, 30) conçue de manière à être appliquée sur le corps d'un être vivant, comprenant un élément de contact corporel (5) pour appliquer l'électrode (1, 2, 3, 4, 30) sur un tissu (12), un élément de préhension manuelle (6) relié à l'élément de contact corporel (5) pour saisir l'électrode (1, 2, 3, 4, 30) et un étranglement (7) se présentant sous la forme d'un ou de plusieurs enroulement(s) pour empêcher l'écoulement de courants de fuite HF à travers l'électrode (1, 2, 3, 4, 30) lors de l'utilisation de l'électrode (1) en combinaison avec un procédé chirurgical HF, dans laquelle l'élément de contact corporel (5) est conçu sous la forme d'une aiguille conductrice de courant à insérer, en particulier une insertion sous-cutanée, dans le tissu (12), et dans laquelle l'étranglement (7) est intégré dans l'élément de préhension manuelle (6).

2. Electrode (1, 2, 3, 4, 30) selon la revendication 1, **caractérisée en ce que** l'étranglement (7) présente une inductance qui est comprise entre 1,0 µH et 100,0 mH, en particulier entre 100,0 µH et 20,0 mH, en particulier entre 1,0 mH et 10,0 mH, en particulier entre 2,0 mH et 9,0 mH, en particulier entre 3,0 mH et 8,0 mH, en particulier entre 4,0 mH et 7,0 mH, et qui est de préférence égale à 4,7 mH ou à 6,8 mH, et/ou **en ce que** l'étranglement (7) est conçu de telle sorte qu'un filtre de bande passante puisse être formé ou soit formé dans le but de générer une fréquence de résonance non supérieure à 400 kHz, en particulier non supérieure à 300 kHz, en particulier non supérieure à 250 kHz, et en particulier non supérieure à 220 kHz.

3. Electrode (1, 2, 3, 4, 30) selon l'une quelconque des revendications 1 ou 2, **caractérisée en ce que** l'électrode (1, 2, 3, 4, 30) est conçue de manière à être utilisée avec un appareil de neurosurveillance peropératoire (9) pour contrôler la capacité de stimulation de fonctions commandées par le système nerveux somatique et/ou végétatif, en particulier pour dériver des signaux neurophysiologiques et/ou stimuler des fonctions commandées par le système nerveux végétatif, de préférence dans des applications chirurgicales en orthopédie, en ORL, en neurochirurgie et/ou en chirurgie générale.

4. Electrode (1, 2, 3, 4, 30) selon l'une quelconque des revendications 1 à 3, **caractérisée en ce que** l'élément de contact corporel (5) est au moins partiellement conducteur et est de préférence constitué d'un métal, en particulier en acier inoxydable, en platine-iridium ou en d'autres métaux, et/ou **en ce que** l'élément de contact corporel (5) présente une longueur qui est comprise entre 3,0 mm et 60,0 mm et/ou un diamètre extérieur qui est compris entre 0,1 mm et 8,0 mm.

5. Electrode (1, 2, 3, 4, 30) selon l'une quelconque des revendications 1 à 4, **caractérisée en ce que** l'élément de préhension manuelle (6) est conçu au moins partiellement sous la forme d'une pièce moulée par injection (10) et/ou d'une gaine thermorétractable (11).

6. Electrode (1, 2, 3, 4, 30) selon l'une quelconque des revendications 1 à 5, **caractérisée en ce que** l'électrode (1, 2, 3, 4, 30) est pourvue de deux ou plus de deux éléments de contact corporel (5), de telle sorte que les éléments de contact corporel (5) soient reliés à une pièce de préhension manuelle (6), et/ou qu'un angle d'environ 90°C soit inclus entre les éléments de contact corporel (5) et l'élément de préhension manuelle (6), en particulier en vue d'une insertion perpendiculaire des éléments de contact corporel (5) dans le tissu, ou de telle sorte que l'axe longitudinal (14) des éléments de contact corporel (5) soit orienté coaxialement à l'axe longitudinal (13) de l'élément de préhension manuelle.

7. Electrode (1, 2, 3, 4, 30) selon l'une quelconque des revendications 1 à 6, **caractérisée en ce que** l'aiguille ou les aiguilles est/sont revêtue(s) ou non revêtue(s), et/ou **en ce que** l'électrode est conçue sous la forme d'une électrode de type tire-bouchon, de préférence dans laquelle l'aiguille présente une forme de tire-bouchon, et de préférence dans laquelle l'électrode (1, 2, 3, 4, 30) est réalisée de façon monopolaire ou bipolaire.
